# EUROPEAN PATENT APPLICATION

(11) **EP 4 512 306 A1**
(43) Date of publication of application: **26.02.2025**
(21) Application number: 24195664.8
(22) Date of filing: 21.08.2024
(51) Int. Cl.: A61B 3/032, A61B 3/113, A61B 3/028

(54) **SIMULTANEOUS EYE-TRACKING CALIBRATION AND VISUAL ACUITY CHECK**

(30) Priority: 25.08.2023 US 202318455664
(71) Applicant: Pixieray Oy, 02630 Espoo (FI)
(72) Inventor: Helin, Emilia, 00810 Helsinki (FI)
(74) Representative: Moosedog Oy

(57) **Abstract**

Disclosed is an optical apparatus (102, 200) comprising eye-tracking means (108, 204); and processor(s) (110, 206) configured to: (a) display image (310, 408A) on screen (112, 304), image representing optotype(s); (b) obtain user input indicative of whether user (302) is able to clearly view optotype (312, 402A) while using optical apparatus; (c) determine whether user is able to clearly view optotype; (d) when user is able to clearly view optotype, collect information of size of optotype, position of optotype and/or optotype features (406), and/or appearance of user's eyes (106) features; (e) when user not able to clearly view optotype, collect information collect information of size of optotype, position of optotype, and/or appearance of user's eyes features; and repeat steps (a) to (e) using next image (408B) representing next optotype(s) (402B) whose size is larger than optotype; and (f) process collected information to determine optical power(s) and calibrate eye-tracking means simultaneously.

## Description

### TECHNICAL FIELD

The present disclosure relates to optical apparatuses incorporating simultaneous eye-tracking calibration and visual acuity check. Moreover, the present disclosure relates to systems incorporating simultaneous eye-tracking calibration and visual acuity check. Furthermore, the present disclosure relates to methods for incorporating simultaneous eye-tracking calibration and visual acuity check.

### BACKGROUND

Eye examinations are conventionally used to screen visual acuity of eyes, and are useful for maintaining health of the eyes and overall well-being. Conventionally, the eye examinations are used to determine a size of an optotype that can be read by a user which is used to determine the visual acuity of the user's eyes, wherein the optotype is displayed on a standardized eye chart and the standardized eye chart is arranged at a predefined distance from the user. Moreover, performing eye examinations regularly helps to at least one of: identify refractive errors in their vision, determine whether the user's eyes suffer from any eye condition, aid in vision development of the user suffering from unidentified eye conditions, monitor the health of the user's eyes suffering from chronic diseases, update prescription for the eyes when optical properties of the eyes change due to any eye condition. Herein, the refractive error occurs when the user suffers from myopia, hypermetropia, astigmatism, and similar. Examples of eye conditions may be glaucoma, cataracts, diabetic retinopathy, and similar. Examples of chronic diseases that could affect the health of the eyes are diabetes, hypertension, and similar.

However, existing techniques and equipment for conducting the eye examinations has several limitations associated herewith. Conventionally, to determine the visual acuity of the user's eyes, the eye examinations are conducted by displaying optotypes of different sizes on a screen (for example, such as the standardized eye chart), wherein the user is asked to read the optotypes using one eye. The test is repeated after switching to another eye. This is time-consuming, and becomes redundant for the user. The eye examinations are also used for screening ocular motility of the user's eyes by conventionally tracing moving calibration dots on the screen using an eye-tracking means, to determine a health of eye muscles of the user's eyes. However, said ocular motility is screened separately for the user's eyes i.e., separate from the screening of the visual acuity for the user's eyes. Moreover, existing techniques and equipment do not consider how well the user can see the calibrations dots, thereby generating incorrect rendering of the user's eyes using conventional rendering algorithms. Therefore, this generates an incorrect calibration data for the eye-tracking means.

Therefore, in light of the foregoing discussion, there exists a need to overcome the aforementioned drawbacks.

### SUMMARY

The aim of the present disclosure is to provide an optical apparatus, a system, and a method for incorporating simultaneous eye-tracking calibration and visual acuity check to facilitate an accurate assessment of the user's eyes, which is iterative in nature. The aim of the present disclosure is achieved by optical apparatuses, systems, and methods for visual acuity check, such methods incorporating simultaneous eye-tracking calibration as defined in the appended independent claims to which reference is made to. Advantageous features are set out in the appended dependent claims.

Throughout the description and claims of this specification, the words "*comprise*", "*include*", "*have*", and "*contain*" and variations of these words, for example "*comprising*" and "*comprises*", mean "*including but not limited to*", and do not exclude other components, items, integers or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

### BRIEF DESCRIPTION OF THE DRAWINGS

FIG. 1 illustrates a block diagram of an architecture of an optical apparatus and a system comprising the optical apparatus, in accordance with an embodiment of the present disclosure;
FIG. 2 illustrates a schematic diagram of an optical apparatus, in accordance with an embodiment of the present disclosure;
FIG. 3 illustrates an environment where a system is being used, such system incorporating simultaneous eye-tracking calibration and visual acuity check, in accordance with an embodiment of the present disclosure;
FIG. 4A shows an exemplary optotype and an exemplary input means, and FIG. 4B shows a flow of an exemplary visual acuity check of a user's eyes conducted using the exemplary optotype and the exemplary input means, in accordance with an embodiment of the present disclosure; and
FIG. 5 illustrates a flowchart illustrating steps of a method incorporating simultaneous eye-tracking calibration and visual acuity check, in accordance with an embodiment of the present disclosure.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In a first aspect, the present disclosure provides an optical apparatus comprising:
eye-tracking means; and
at least one processor configured to:
   (a) display a given image on a screen, the given image representing at least one optotype;
   (b) obtain a given user input indicative of whether the user is able to clearly view a given optotype represented in the given image while using the optical apparatus;
   (c) determine, based on the given user input, whether the user is able to clearly view the given optotype represented in the given image;
   (d) when it is determined that the user is able to clearly view the given optotype, collect information indicative of at least one of: a size of the given optotype that the user is able to clearly view, a position of the given optotype and/or optotype features in the given image, an appearance of features of the user's eyes when the user is able to clearly view the given optotype that is displayed at said position;
   (e) when it is determined that the user is not able to clearly view the given optotype,
      collect information indicative of at least one of: a size of the given optotype that the user is not able to clearly view, a position of the given optotype and/or optotype features in the given image, an appearance of the features of the user's eyes when the user is not able to clearly view the given optotype that is displayed at said position; and
      repeat steps (a) to (e) using a next image, wherein the next image represents at least one next optotype whose size is larger than a size of the given optotype that was represented in the given image; and
   (f) process the collected information to determine one or more optical powers specific to the user and to calibrate the eye-tracking means simultaneously.

The aforementioned optical apparatus incorporates simultaneous eye-tracking means calibration and visual acuity check. Herein, optotypes of different sizes at different positions are displayed to the user on the screen, to collect information regarding if the user can clearly view a given optotype and determine movement of the user's eyes when viewing the given optotype at the different positions. This information is then used to dynamically determine the one or more optical powers specific to the user, whilst simultaneously calibrate the eye-tracking means dynamically to establish a precise mapping between the movement of the user's eyes and corresponding position of the given optotype. This makes the calibration process simple yet effective. The one or more optical powers can then be used to improve a visual experience of the user, by producing at least one of the one or more optical powers at an active optical element of the optical apparatus (or another optical apparatus). Beneficially, the active optical element can easily be implemented in various types of optical apparatuses, for example, such as a pair of glasses, a pair of sunglasses, smart glasses, a head-mounted display, or similar. A synergistic effect of the aforementioned features is that the visual acuity check and the calibration of the eye-tracking means are carried out simultaneously, thereby reducing processing resources, time, and cost required. When the user is either able to clearly view the given optotype or not able to clearly view the given optotype, information related to the given optotype and the user's eyes when viewing such given optotype is collected to analyse performance of the eyes. The size of the given optotype is changed when the visual acuity check is repeated, so that an extent of the visual acuity of the user's eyes can be determined. This aids in determining the one or more optical powers accurately and precisely.

In a second aspect, the present disclosure provides a system comprising:
the optical apparatus according to the first aspect;
the screen that is to be employed to display images; and
at least one input means that is to be employed to obtain user input.

The aforementioned system incorporates simultaneous eye-tracking means calibration and visual acuity check. Herein, the images representing optotypes are displayed on the screen, and the user provides an input using the at least one input means, based on whether the user is able to clearly view the given optotype. A synergistic effect of the aforementioned features is that while using the optical apparatus this data can be used to simultaneously do a visual acuity check and calibrate eye-tracking means. Such screens and the at least one input means are well-known in the art, and do not require additional time or cost for their configuration.

In a third aspect, the present disclosure provides a method implemented by an optical apparatus comprising an eye-tracking means and at least one processor, wherein the method comprises:
(a) displaying a given image on a screen, the given image representing at least one optotype;
(b) obtaining a given user input indicative of whether the user is able to clearly view a given optotype represented in the given image while using an optical apparatus according to the first aspect;
(c) determining, based on the given user input, whether the user is able to clearly view the given optotype represented in the given image;
(d) when it is determined that the user is able to clearly view the given optotype, collecting information indicative of at least one of: a size of the given optotype that the user is able to clearly view, a position of the given optotype and/or optotype features in the given image, an appearance of features of the user's eyes when the user is able to clearly view the given optotype that is displayed at said position;
(e) when it is determined that the user is not able to clearly view the given optotype,
   collecting information indicative of at least one of: a size of the given optotype that the user is not able to clearly view, a position of the given optotype and/or optotype features in the given image, an appearance of the features of the user's eyes when the user is not able to clearly view the given optotype that is displayed at said position; and
   repeating steps (a) to (e) using a next image, wherein the next image represents at least one next optotype whose size is larger than a size of the given optotype that was represented in the given image; and
(f) processing the collected information to determine one or more optical powers specific to the user and to calibrate the eye-tracking means simultaneously.

The aforementioned method incorporates simultaneous eye-tracking means calibration and visual acuity check. Herein, displaying optotypes of different sizes at different positions on the screen enables collecting information regarding a size of the optotype that the user can view and determining how flexibly the user's eye moves when viewing said optotypes at the different positions. This information can be used for calibrating the eye-tracking means for each of the different optotypes displayed to the user. A synergistic effect of the aforementioned features is that the visual acuity check and the calibration of the eye-tracking means are carried out simultaneously, thereby reducing processing resources, time, and cost required. When the user is either able to clearly view the given optotype or not able to clearly view the given optotype, information related to the given optotype and the user's eyes when viewing such given optotype is collected to analyse performance of the eyes. The steps of the method are repeated by displaying optotypes of different sizes to the user, so that an extent of the visual acuity of the user's eyes can be determined. This aids in determining the one or more optical powers accurately and precisely. Therefore, the method described herein is simple, robust, fast, reliable, and easy to implement.

Throughout the present disclosure, the term "*optical apparatus*" refers to an apparatus that is to be used by the user to perform the visual acuity check and the aforesaid calibration simultaneously. In some implementations, the optical apparatus may be a wearable apparatus that is to be worn over the eyes of the user. Examples of such an optical apparatus include, but are not limited to, a pair of glasses, a pair of sunglasses, a pair of smart glasses, and a head-mounted display. In other implementations, the optical apparatus may be a non-wearable apparatus. As an example, in such an implementation, the optical apparatus may be positioned on a table that is kept in front of the user.

In implementations where the optical apparatus is a wearable, the optical apparatus optionally comprises an active optical element per eye. Throughout the present disclosure, the term "*active optical element*" refers to an optical element having one or more optical parameters that can be controlled, e.g. are actively (i.e., dynamically) adjustable.

Throughout the present disclosure, the term "*eye-tracking means*" refers to a specialized equipment that is employed to at least one of: detect a direction of gaze of the user of the optical apparatus, follow a direction of gaze of the user of the optical apparatus, monitor features of the user's eyes when the user uses the optical apparatus. Such features may comprise at least one of: a shape of a pupil of the user's eye, a size of the pupil, corneal reflections of light emanating from a real-world environment from a surface of the user's eye, a relative position of the pupil with respect to the corneal reflections, a relative position of the pupil with respect to corners of the user's eye. The eye-tracking is performed when the optical apparatus, in operation, is used by the user.

Optionally, the eye-tracking means is implemented by way of at least one of: contact lenses having sensors, cameras monitoring features of the user's eyes. Such eye-tracking means are well-known in the art. The eye-tracking means is configured to collect eye-tracking data and send the eye-tracking data to the at least one processor. It will be appreciated that the eye-tracking data is collected repeatedly by the eye-tracking means throughout an operation of the optical apparatus, as the gaze and the features of the user's eyes keeps changing whilst she/he uses the optical apparatus. An up-to-date eye-tracking data (indicative of gaze directions and the features of the user's eyes) allows for adaptively controlling the active optical element of the optical apparatus in an accurate manner, for effectively checking the visual acuity of the user's eyes. In an instance, when the eye-tracking means is implemented as the camera, the eye-tracking data is in form of images of the user's eyes. In another instance, when the eye-tracking means is implemented by way of contact lenses having sensors, the eye-tracking data is sensor data collected from the sensors.

It will be appreciated that the at least one processor is communicably coupled to the eye-tracking means and optionally the active optical element. The at least one processor could be implemented as any one of: a microprocessor, a microcontroller, or a controller. As an example, the at least one processor could be implemented as an application-specific integrated circuit (ASIC) chip or a reduced instruction set computer (RISC) chip.

The given image is a visual representation of the at least one optotype at a particular position. In other words, the given image encompasses shape information and/or colour information of the at least one optotype, and optionally other attributes associated with the at least one optotype (for example, such as depth information, luminance information, transparency information, and the like). Optionally, the given image is a visible-light image. Herein, the visible-light image is composed using Red-Green-Blue (RGB) spectrum. Optionally, the given image is a high-quality (i.e., high-resolution) image. The given image could be in a JPEG format, a JPG format, a PNG format, and the like. The given image is displayed to the user to assess her/his ability to recognize and identify the at least one optotype accurately.

Throughout the present disclosure, the term "*optotype*" refers to a standardized number, a standardized alphabet character, and/or a standardized symbol, of a particular size and at a particular position in the given image used for the visual acuity check of the user's eyes, and facilitating maintaining consistency when conducting the visual acuity check across different medical professionals, hospitals, and/or optical apparatuses. Herein, the particular size of the at least one optotype is used for determining a visual acuity of the user's eyes, and the particular position of the at least one optotype is used to assess an ability of the user's eyes to track the at least one optotype and calibrate the eye-tracking means. Herein, when the screen is at a predefined distance (as is described later) from the user, the particular size of the at least one optotype corresponds to a particular level of visual detail of the at least one optotype which the user can accurately identify. This particular level of the visual detail could be represented in a form of a ratio between the predefined distance and a distance at which another user with a normal eyesight (i.e., not suffering from any eye conditions) is able to clearly view the at least one optotype displayed on the screen. Furthermore, the particular position of the at least one optotype or the optotype features corresponds to a relative position of the at least one optotype with respect to at least one corner of the screen where it is displayed and/or to a relative position of the optotype features with respect to an entirety of the at least one optotype. Herein, the term "*optotype features*" refers to any discernible gap and/or a discernible mark that is present within the given optotype, that the user needs to clearly view so that the visual acuity of the user's eyes can be determined. It will be appreciated that the at least one optotype has a high contrast with respect to its background to enhance visibility. Examples of the at least one optotype being the standardized number may include, but are not limited to, at least a single digit number, a decimal notation, and a sequence of numbers. Examples of the at least one optotype being the standardized alphabet character may include, but are not limited to, E, C, H, O, N, D, A, and T. Examples of the at least one optotype being the standardized symbol may include, but are not limited to, a ring, a circle, a square, a cross, a triangle, a diamond, and a star. The at least one optotype can be dynamically selected by the medical professional and/or the user.

Optionally, the at least one optotype comprises at least one of: a Landolt C optotype, a Snellen optotype, a Sloan optotype, an Early Treatment Diabetic Retinopathy Study (ETDRS) optotype, an HOTV letter, an Allen figure, an alphabet character (for example, such as the standardized alphabet character), a number (for example, such as the standardized number), a symbol (for example, such as the standardized symbol), a design, a freeform shape. The Landolt C optotype comprises any one of: a ring, a circle with an optotype feature (i.e., a predefined gap) at a particular position (for example, such as a top position, a bottom position, a left position, a right position) within any one of: the ring, the circle. Beneficially, the Landolt C optotype could be used for at least one of: when the user is not familiar with the number and/or the alphabet character, when the user is not able to differentiate between the number and/or the alphabet character, when individuals have cognitive impairments or visual impairments. The Snellen optotype, the ETDRS optotype, and the HOTV optotype comprise the standardized numbers and/or the standardized alphabet characters in uppercase, wherein said standardized numbers and/or the standardized alphabets are arranged in rows and columns, with their size decreasing from top to bottom. The ETDRS optotype comprises a particular number of strokes and a standardized spacing between each of the standardized numbers and/or the standardized alphabet characters, and is thus displayed to the user suffering from any eye condition, for example, such as diabetic retinopathy. The HOTV letters comprises only the standardized alphabet characters H, O, T, and V. The Allen figure comprises a standardized shape that is designed to be easily recognisable by the user who is unable to read or recognize the standardized number and/or the standardized alphabet character. The design comprises at least one of: the standardized alphabet character, the standardized number, the standardized symbol, a decorative pattern, which can be created by the medical professional based on a medical history of the user or can be predesigned. The freeform shapes are unique and distinct patterns. Beneficially, the freeform shapes are used when the user is unable to recognize the standardized alphabet character, the standardized numbers, and the standardized symbols.

The at least one processor is communicably coupled with the screen. Herein, the term "*screen*" refers to a physical surface (for example, such as a wall, a projection screen) or a monitor (for example, such as a television monitor, a computer monitor, a smartphone display, and similar) on which the given image is displayed. In an embodiment, the screen is integrated with a display device, wherein the at least one processor is communicably coupled with the display device. In such embodiments, the screen is directly coupled to hardware and/or software components of the display device. Examples of such display devices may include, but are not limited to, cell phones, tablets, phablets, computers, laptops, personal digital assistants, smart watches, and television. In another embodiment, the screen is separate from the display device, wherein the at least one processor is communicably coupled with the display device in a wired manner and/or a wireless manner. In such implementations, the screen is indirectly coupled to hardware and software components of the display device. Examples of such display devices may include, but are not limited to, a projector, a display of an external monitor.

Optionally, the user views the screen from a predefined distance (D1) lying in a range of 25 centimetres to 750 centimetres. The predefined distance (D1) lies in a range of 25, 50, 65, 85, 160, 320, 480, 640 or 700 centimetres to 40, 55, 75, 95, 190, 380, 570, 690, or 750 centimetres. A technical benefit of arranging the screen at the predefined distance from the user is that measurements from conducting the visual acuity check of the user's eyes can be standardized across different users and different sessions for visual acuity check. In an instance, for determining the visual acuity of the user for near vision, the predefined distance (D1) lies in a range of 35 centimetres to 40 centimetres. In another instance, for determining the visual acuity of the user for intermediate vision, the predefined distance (D1) lies in a range of 50 centimetres to 75 centimetres.

Throughout the present disclosure, the term "*given user input*" refers to a response and/or feedback provided by the user after the given image is viewed by the user via at least one input means (as described later) and received by the at least one processor, upon perceiving the given optotype with clarity and accuracy, when the optical apparatus is in use. Optionally, the given user input is provided by the user in a form of a voice message, a text message, a haptic feedback, a touch feedback, a gesture, a movement, and similar.

Optionally, the given user input is obtained via at least one input means communicably coupled to the at least one processor. Optionally, the at least one input means is communicably coupled to the at least one processor in a wired manner and/or a wireless manner. The at least one input means could comprise at least one of: a touch-sensitive surface, a button, a keyboard, a mouse, an XR controller, an audio sensor, a remote controller, a physical slider, a microphone, a recorder. As an example, the input means can be implemented as a touch-sensitive surface of a smartphone, wherein a processor of the smartphone is configured to execute a software application to provide the user with a user interface on which the user can provide the given user input. The at least one input means provides a flexibility to the user using the optical apparatus by allowing the user to provide the given input as and when the user is able to clearly see the given optotype. A technical effect of communicably coupling the at least one input means to the at least one processor is that the given user input is transmitted accurately to the at least one processor for at least one of: analysis, interpretation, action to be performed.

In a first example, when the optical apparatus is in use, a given image may be displayed on the screen. Herein, the given image may represent the Landolt C optotype. The user may view the screen from a predefined distance of 300 centimetres. The user may provide an input using the at least one input means, wherein the at least one input means may be a remote controller comprising four buttons namely, an up-direction button, a down-direction button, a left-direction button, and a right-direction button that may correspond to an optotype feature being positioned at the top position, the bottom position, the left position, and the right position, respectively within the Landolt C optotype. In the given image, the optotype feature may be located at the left position within the Landolt C optotype. In an instance, the given user input may be provided by the user by pressing the up-direction button when the user is not able to clearly view the optotype feature in the right position within the Landolt C optotype. Alternatively, in another instance, the given user input may be provided by the user by pressing the left-direction button of the remote controller when the user is able to clearly view the optotype feature in the left position within the Landolt C optotype.

Optionally, the optical apparatus further comprises:
the active optical element per eye;
a frame employed to hold the active optical element per eye; and
at least one input means, mounted on a temple of the frame, that is to be used by the user for providing the given user input.

In this regard, the frame is designed in a manner that the active optical element is firmly arranged on the frame. In an example, when the optical apparatus is implemented as a pair of eyeglasses, the frame may hold two active optical elements, wherein a first active optical element is employed for a first eye of the user, and a second active optical element is employed for the second eye of the user. It will be appreciated that a material of the frame could be plastic, metal, polymer, and the like. Moreover, the frame may be lightweight, ergonomically designed, and easy to use.

The at least one input means mounted on a temple of the frame provides a flexibility to the user using the optical apparatus by providing the given user input by conveniently one of: the physical slider, the button mounted on the temple of the frame itself. A technical benefit of mounting the at least one input means on the temple of the frame is that it renders hands of the users free and enables the user to conveniently interact with a surrounding environment. For example, the at least one input means may be two physical sliders mounted on the temple of the frame. Herein, a first physical slider may be mounted on a first temple of the frame, and a second physical slider may be mounted on a second temple of the frame. The first physical slider may be used when the user is able to clearly view the given optotype, and the second physical slider may be used when the user is not able to clearly view the given optotype.

The at least one processor is configured to process the given user input using at least one processing algorithm based on the form of the given user input obtained by the user. A technical effect is that the given user input is verified where it is determined whether the user is able to correctly identify the given optotype. Examples of the at least one processing algorithm may include, but are not limited to, tokenization algorithm, Named Entity Recognition, Text classification algorithms, signal processing algorithms, natural language processing, support vector machines, and deep neural networks.

When the at least one processor is configured to determine that the user is able to clearly view the given image, the information regarding the size of the given optotype represented in the given image at that instant is collected. A technical benefit of collecting the information is that it helps to assess a level of the visual acuity of the user's eyes, and further provide a quantitative measure of their vision. Herein, based on the size of the given optotype, the visual acuity is quantitatively measured in a form of a ratio of the predefined distance at which the given optotype is presented to the size of the given optotype that the user can clearly view. Optionally, the size of the given optotype comprises at least one of: a length, a height, a width, a spacing of the given optotype. The size of the given optotype corresponds to acuteness of the user's eyes, and allows to evaluate the visual acuity at different levels of visual acuity. Such different levels of visual acuity are achieved by incrementally increasing or incrementally decreasing the size of the given optotype, beneficially allowing for a precise assessment of the visual acuity. This precise assessment is beneficial when distinguishing between small changes in vision of the user at different levels of the of visual acuity.

When the user is able to clearly view the given optotype represented in the given image, an appearance of the features of the user's eyes changes with respect to the given optotype. Such features may comprise at least one of: a position of the pupil of the user's eyes, the shape of the pupil of the user's eyes, a tension of an eyelid of the user's eyes, an extent of opening of the user's eyes, an extent of raising of eyebrows, an extent of squinting of the user's eyes. The information indicative of the features of the user's eyes is collected using the eye-tracking means when the optical apparatus, in operation, is used by the user. Such eye-tracking means has been described in detail above. The eye-tracking means is configured to collect the information indicative of the appearance of features of the user's eyes and send the information to the at least one processor. It will be appreciated that said information is collected repeatedly by the eye-tracking means throughout the operation of the optical apparatus, as the appearance of the features of the user's eyes keeps changing whilst she/he uses the optical apparatus.

When the user is not able to clearly view the given image, the information collected at such an instance is distinctly different from the information collected when the user is able to clearly view the given image. A technical benefit of collecting said information is that subsequently, with the help of the information collected by the eye-tracking means, the optical apparatus is able to recognize when the user is not able to clearly view the given optotype, and accordingly remind the user to repeat the visual acuity. Herein, the information is collected in a manner similar to the manner described above.

When the user is not able to clearly view the given image, the visual acuity check is continued, and the at least one processor is configured to display the next image to the user. Herein, the at least one next optotype could be at least one of: of a different size than the size of the given optotype, at a different position on the screen than the given optotype, with a different optotype feature than the given optotype. The next image is a visual representation of the at least one next optotype at a particular position. Similar to the given image, the next image is a visible-light image. The next image is a high-quality (i.e., high-resolution) image. The next image could be in a JPEG format, a JPG format, a PNG format, and the like.

Herein, a technical effect of displaying the at least one next optotype which is larger than the size of the given optotype is to determine a maximum size of the given optotype that the user can clearly view. Herein, the size of the at least one next optotype is made larger than the size of the given optotype by any one of: dynamically increasing the size of the given optotype using an adaptive algorithm, manually increasing the size of the given optotype by a person (for example, such as a medical professional) determining the visual acuity of the user, incrementally increasing the size of the given optotype by the at least one processor by employing a function or a rule. Herein, the adaptive algorithm is used to determine an appropriate size of the at least one next optotype in the next image based on the given user input.

Optionally, the at least one processor is configured to, when it is determined that the user is able to clearly view the given optotype, repeat steps (a) to (e) using another next image, wherein the another next image represents at least one other next optotype whose size is smaller than the size of the given optotype that was represented in the given image. A technical effect of displaying the at least one other next optotype which is smaller than the size of the given optotype is to determine a minimum size of the given optotype that the user can clearly view. The size of the at least one other next optotype is made smaller than the size of the given optotype similarly as described above for increasing the size of the given optotype.

In some implementations, the one or more optical powers to be determined comprises a single optical power. In other implementations, the one or more optical powers to be determined comprises a plurality of optical powers. It will be appreciated that the plurality of optical powers could be produced in different regions of the active optical element at a same time, for example, for enabling the user to focus on the at least one other next optotype whose size is smaller than the size of the given optotype (using a negative optical power or a zero optical power, depending on the user's need) and to focus on the at least one next optotype whose size is larger than the size of the given optotype (using a zero optical power or a positive optical power, depending on the user's need). Optionally, in this regard, the one or more optical powers is at least one of: the positive optical power, the negative optical power, the zero optical power. Herein, the positive optical power is used for reading or focussing on the at least one next optotype whose size is larger than the size of the given optotype. The negative optical power is used for focussing on the at least one other next optotype whose size is smaller than the size of the given optotype. The zero optical power may be used when the user does not have myopia and needs to focus on distant objects, or when the user does not have hypermetropia or presbyopia and needs to focus on nearby objects.

Simultaneously, the at least one processor is configured to calibrate the eye-tracking means by employing calibration techniques based on the processed collected information. In particular, calibration parameters of the eye-tracking means are estimated using the collected information. Then, these calibration parameters are used to correct any distortion in the eye-tracking means. Herein, the calibration parameters describe a mathematical relationship between the collected information and the eye-tracking means. Optionally, when determining the calibration parameter, the at least one processor employs any function, a rule, and/or a mathematical formula. Such functions, rules, mathematical formulas are well-known in the art.

Optionally, the optical apparatus further comprises the active optical element per eye, wherein the at least one processor is configured to:
control the active optical element to produce at least one of the one or more optical powers;
repeat steps (a) to (e); and
process newly-collected information to further calibrate the eye-tracking means.

A technical effect of further calibrating the eye-tracking means in such a manner is that it is ensured that the eye-tracking means takes into account variations in optical properties of the user's eyes based on the at least one of the one or more optical powers produced. Furthermore, by repeating the steps (a) to (e), an accuracy of calibration of the eye-tracking means could be verified. In this regard, the at least one processor is configured to generate a control signal to control the active optical element, based on how clearly the user is able to see the given image representing the given optotype. The control signal could be a voltage signal and/or a current signal. It will be appreciated that the drive signal for the active optical element for each may be different. The at least one processor produces the at least one of the one or more optical powers that was determined when processing the collected information. In order to verify whether the production of the given optical power (namely, the at least one of the one or more optical powers) matches the calibration of the eye-tracking means, and vice versa, the visual acuity check is repeated by displaying the given image to the user. Such matching is determined when the user is able to clearly view the given optotype in the given image, and experiences no difficulty or blurred vision while viewing said given optotype. Continuing in reference with the first example, when the user is not able to clearly view the given optotype in the given image, the at least one processor may be configured to control the active optical element to produce an optical power of +1.5 dioptres. The visual acuity check may be repeated, which may generate new information which may be collected. This new information may be used to calibrate the eye-tracking means. The visual acuity check may be further repeated to verify that the optical power of +1.5 dioptres produced matches the calibration of the eye-tracking means.

Optionally, the at least one processor is configured to process the newly-collected information. Herein, when the user is able to clearly view the given optotype, the newly-collected information is indicative of at least one of: the size of the given optotype that the user is able to clearly view, the position of the given optotype and/or given optotype features in the given image, the appearance of features of the user's eyes when the user is able to clearly view the given optotype that is displayed at said position. Alternatively, when the user is not able to clearly view the given optotype, the newly-collected information is indicative of at least one of: the size of the given optotype that the user is not able to clearly view, the position of the given optotype and/or given optotype features in the given image, the appearance of features of the user's eyes when the user is not able to clearly view the given optotype that is displayed at said position.

Subsequently, the at least one processor is optionally configured to further calibrate the eye-tracking means by employing the calibration techniques using the processed newly-collected information. In particular, other calibration parameters of the eye-tracking means are estimated using the newly-collected information. Then, these other calibration parameters are used to correct any further distortion in the eye-tracking means.

Optionally, the at least one processor is configured to adjust the at least one of the one or more optical powers, when it is determined that the user is not able to clearly view a given optotype. A technical effect of adjusting the at least one of the one or more optical powers is that it ensures that the user's vision is corrected in a precise manner, which enables the user to experience a clearer and a sharper vision and/or reduced eyestrain. In this regard, even after producing the given optical power, the user may have difficulty in viewing the given optotype. Herein, the given optical power could be in close proximity to an optical power (that lies in the at least one of the one or more optical powers) required so that the user is able to clearly view the given optotype. Hence, a magnitude of the optical power (irrespective of sign) is increased if more optical power is needed than the given optical power. Alternatively, a magnitude of the optical power (irrespective of sign) is decreased if less optical power is needed than the given optical power. Continuing in reference with the first example, steps (a) to (e) are repeated when the user uses the optical apparatus of the optical power +1.5 dioptres, when the at least one processor may determine that the user is still not able to clearly view the next optotype. Hence, the optical power may be adjusted by +/-0.5 dioptres, which increases the optical power of the active optical element to +2.0 dioptres.

Optionally, the optical apparatus further comprises the active optical element per eye, wherein the at least one processor is further configured to:
process eye-tracking data, collected by the eye-tracking means, to determine at least one of: gaze directions of the user's eyes, a gaze point of the user;
determine a given optical depth at which the user is gazing, based on at least one of: an angle of convergence of the gaze directions of the user's eyes, an interpupillary distance of the user's eyes, the gaze point of the user; and
select at least one of the one or more optical powers to be produced by the active optical element, based on the given optical depth at which the user is gazing.

In this regard, the term "*gaze direction*" refers to a direction in which the user's eyes is gazing. The gaze direction may be represented by a gaze vector. The "*gaze point*" is a point in the real-world environment at which the user's gaze is focused. The gaze point is a point of interest of the user, in the real-world environment. Optionally, the gaze point is determined by mapping the gaze directions of the user's eyes to a corresponding point in the real-world environment, at which the gaze directions converge. Optionally, when processing the eye-tracking data, the at least one processor is configured to employ at least one of: an image processing algorithm, a feature extraction algorithm, a data processing algorithm. Other suitable algorithm(s) can also be employed.

The term "*optical depth*" refers to an optical distance between the point of interest and the optical apparatus used by the user. Optionally, the at least one processor is configured to determine the optical depth, when the gaze directions of the user's eyes converge at any portion of the screen. Hence, the "*angle of convergence*" is an angle at subtended by the gaze directions of the user's eyes, when gazing at any portion of the screen. Optionally, the at least one processor is configured to use triangulation for calculating the inter-pupillary distance of the user. Herein, the inter-pupillary distance of the user's eyes can be an average inter-pupillary distance. It will be appreciated that when the angle of convergence of the gaze directions of the user's eyes, the interpupillary distance of the user's eyes, the gaze point of the user are already known to the at least one processor, the given optical depth at which the user is gazing can be easily determined by the at least one processor, for example, using at least one mathematical technique. The at least one mathematical technique could be at least one of a triangulation technique, a geometry-based technique, a trigonometry-based technique. Determining the gaze convergence distance, based on the convergence of the gaze directions is well-known in the art.

Herein, the at least one of the one or more optical powers may be the negative optical power, the zero optical power, or the positive optical power, as already described above. Furthermore, in this regard, the one or more optical powers are different optical powers corresponding to different optical depths. The at least one of the one or more optical powers is selected from amongst the one or more optical powers (that may be prescribed for the user for the different optical depths), based on the optical depth at which the user is looking. The at least one of the one or more optical powers selected for may be a first eye may be same as or different from the at least one of the one or more optical powers selected for a second eye of the user.

The present disclosure also relates to the second aspect as described above. Various embodiments and variants disclosed above, with respect to the aforementioned first aspect, apply *mutatis mutandis* to the second aspect.

Optionally, the screen is one of: a display of a device of the user or a medical professional, a projection surface of a projector. Herein, the term "*device*" refers to a communication device which is operable to display a given image representing the given optotype, when the optical apparatus is in use. A technical effect of screen is that it facilitates dynamic presentation of a given image representing a given optotype to the user. Such dynamic presentation allows the user or the medical professional to at least one of: change a size, change a position, change an orientation, of the given optotype during the visual acuity check. When the screen is the display of the device of the user, that means that the device belongs and used by the user herself/himself. In this regard, the user optionally interacts with the display for viewing a given image, providing a given user input to convey whether the user is able to clearly view the given optotype represented in the image. When the screen is the display of the medical professional, that means that the device belongs to the medical professional. Examples of such medical professionals may include, but are not limited to, an eye care specialist, ophthalmologist, and optometrist. The device is optionally used for conducting the visual acuity check of the user's eyes, diagnostic tests, or displaying medical information relevant to the visual acuity of the user's eyes. Examples of the device may include, but are not limited to, a laptop, a computer, a tablet, a phablet, a cellular phone, a smartphone, and a smartwatch. Examples of the display may include, but are not limited to, a television display, a monitor, a laptop screen, and a smartwatch display.

Furthermore, the projector is used to project the given image representing the given optotype using rays of light emanating from a light source, onto the projection surface. Herein, the projection surface is used to display the given image representing the given optotype, when the optical apparatus is in use. A material of the projection surface should be such that the rays of light can be reflected or diffused to form the given image in an accurate manner.

In a second example, there may be an exemplary environment where the system for incorporating simultaneous eye-tracking calibration and visual acuity check is used. The environment may include the system, and a user. In the environment, the system may be used for conducting a visual acuity check of eyes of the user, and for simultaneously performing the eye-tracking calibration. The system may comprise the optical apparatus (for example, such as a pair of eyeglasses), the screen (for example, such as a television display), and at least one input means (for example, such as a remote controller). The user may view the television display from a predefined distance D1 of, for example, 300 centimetres, when the test is conducted. During the visual acuity check, a given image may be displayed on the television display, wherein the given image may represent a given optotype (for example, such as a symbol). A given user input may be provided by the user using the remote controller, wherein the given user input may be indicative of whether the user is able to clearly view the symbol that may be represented in the given image while using the pair of eyeglasses. Subsequently, based on the given user input, it may be determined whether the user is able to clearly view the symbol that may be represented in the given image.

The present disclosure also relates to the third aspect as described above. Various embodiments and variants disclosed above, with respect to the aforementioned first aspect, apply *mutatis mutandis* to the third aspect.

Optionally, when it is determined that the user is able to clearly view the given optotype, repeating steps (a) to (e) using another next image, wherein the another next image represents at least one other next optotype whose size is smaller than the size of the given optotype that was represented in the given image.

Optionally, the method comprises:
controlling an active optical element of the optical apparatus to produce at least one of the one or more optical powers;
repeating steps (a) to (e); and
processing newly-collected information to further calibrate the eye-tracking means.

Optionally, the method comprises adjusting the at least one of the one or more optical powers, when it is determined that the user is not able to clearly view a given optotype.

Optionally, the method further comprises:
processing eye-tracking data, collected by the eye-tracking means, to determine at least one of: gaze directions of the user's eyes, a gaze point of the user;
determining a given optical depth at which the user is gazing, based on at least one of: an angle of convergence of the gaze directions of the user's eyes, an interpupillary distance of the user's eyes, the gaze point of the user; and
selecting at least one of the one or more optical powers to be produced by an active optical element of the optical apparatus, based on the given optical depth at which the user is gazing.

### DETAILED DESCRIPTION OF THE DRAWINGS

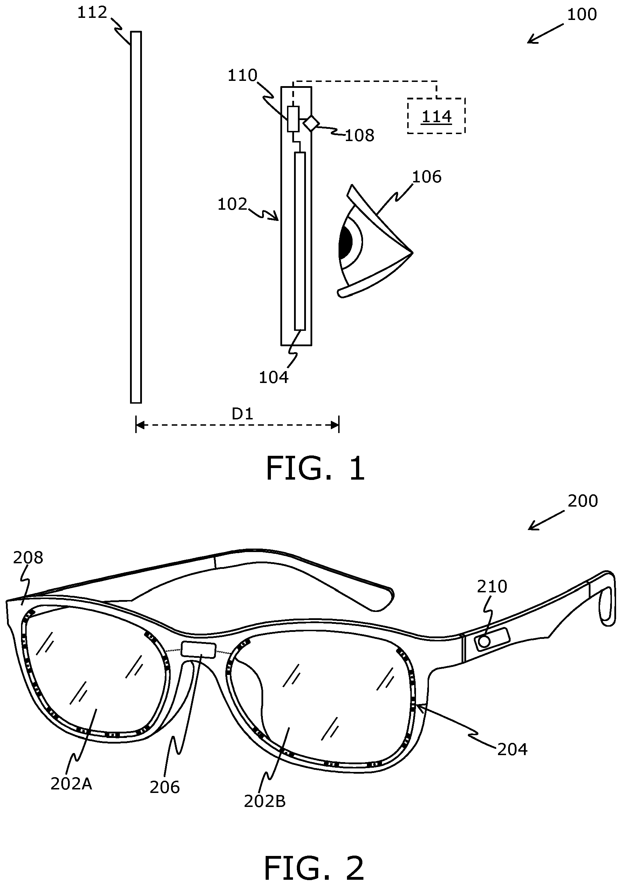

Referring to FIG. 1, there is illustrated a block diagram of an architecture of an optical apparatus **102** and a system **100** comprising the optical apparatus **102,** in accordance with an embodiment of the present disclosure. The optical apparatus **102** comprises an eye-tracking means **108** and at least one processor (depicted as a processor **110**). Optionally, the optical apparatus also comprises an active optical element **104** per eye **106.** The processor **110** is communicably coupled to the active optical element **104** and the eye-tracking means **108.** The processor **110** is configured to perform various operations, as described earlier with respect to the aforementioned first aspect.

Apart from the optical apparatus **102,** the system also comprises a screen **112.** Optionally, when the optical apparatus **102** is in use, a user views the screen **112** from a predefined distance **D1.** The system further comprises at least one input means (depicted as an input means **114**). The processor **110** is further communicably coupled with the input means **114.**

FIG. 1 is merely an example, which should not unduly limit the scope of the claims herein. A person skilled in the art will recognize many variations, alternatives, and modifications of embodiments of the present disclosure. For example, the eye-tracking means **108** could be implemented as separate eye-tracking means per eye.

Referring to FIG. 2, illustrated is a schematic diagram of an optical apparatus **200,** in accordance with an embodiment of the present disclosure. As shown, the optical apparatus **200** is implemented as a pair of eyeglasses. The optical apparatus **200** comprises an eye-tracking means **204,** at least one processor (depicted as a processor **206**), a frame **208** employed for holding the active optical element **202A** for the right eye and the active optical element **202B** for the left eye, and at least one input means (depicted as input means **210**) mounted on the frame **208.** Optionally, the optical apparatus **200** also comprises an active optical element per eye (depicted as an active optical element **202A** for a right eye and an active optical element **202B** for a left eye). The processor **206** is communicably coupled with the active optical elements **202A-B** and the eye-tracking means **204.** The input means **210** can be implemented, for example, as a physical sliders or a button. The input means **210** can be used by the user for providing a given user input. In an exemplary scenario, a given image may be displayed on a screen for visual acuity check of a user's eyes, wherein the given image represents a given optotype. The input means **210** may be used by the user to indicate whether the user is able to clearly view the given optotype represented in the given image while using the optical apparatus **200.**

FIG. 2 is merely an example, which should not unduly limit the scope of claims therein. It is to be understood that the specific implementation of the optical apparatus **200** is provided as an example and is not to be construed as limiting it to specific numbers or types of active optical elements, components of the eye-tracking means, and input means. A person skilled in the art will recognize many variations, alternatives, and modifications of embodiments of the present disclosure.

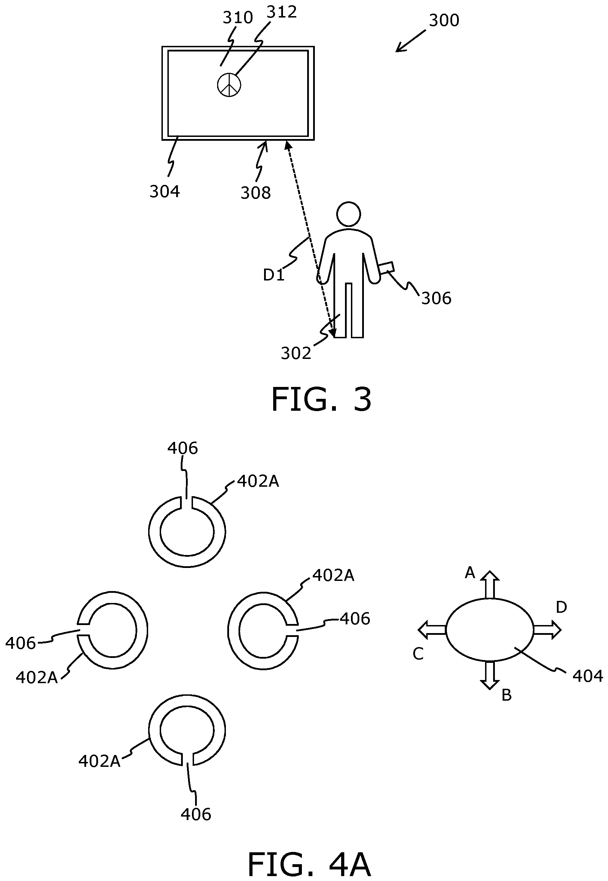

Referring to FIG. 3, there is illustrated an environment **300** where a system is being used, such system incorporating simultaneous eye-tracking calibration and visual acuity check, in accordance with an embodiment of the present disclosure. The environment **300** includes the system, and a user **302.** A test is conducted for assessing a visual acuity of the user's eyes in this environment **300.** The system comprises an optical apparatus (that is used by the user **302** during the simultaneous eye-tracking calibration and visual acuity check), a screen **304,** and at least one input means (depicted as an input means **306**). The screen **304** is depicted as a display of a device **308** of the user **302.** The user **302** views the screen **304,** from a predefined distance **D1** (for example, such as 300 centimetres), during the visual acuity check. During the visual acuity check, a given image **310** is displayed on the screen **304,** wherein the given image **310** represents at least one optotype (depicted as a given optotype **312,** wherein the given optotype **312** is depicted as a symbol). A given user input is provided by the user **302** using the input means **306,** wherein the given user input is indicative of whether the user **302** is able to clearly view the given optotype **312** represented in the given image **310** while using the optical apparatus. Upon determining that the user **302** is either able to or not able to clearly view the given optotype **312,** information is collected indicative of: a size of the given optotype **312** that the user is either able to or not able to clearly view, a position of the given optotype **312** in the given image **310,** an appearance of the features of the user's eyes when the user is either able to or not able to clearly view the given optotype **312** that is displayed at said position. Based on this information procured while conducting the visual acuity check using the system, the eye-tracking means is calibrated, while simultaneously determining one or more optical powers specific to the user.

FIG. 3 is merely an example, which should not unduly limit the scope of the claims herein. A person skilled in the art will recognize many variations, alternatives, and modifications of embodiments of the present disclosure.

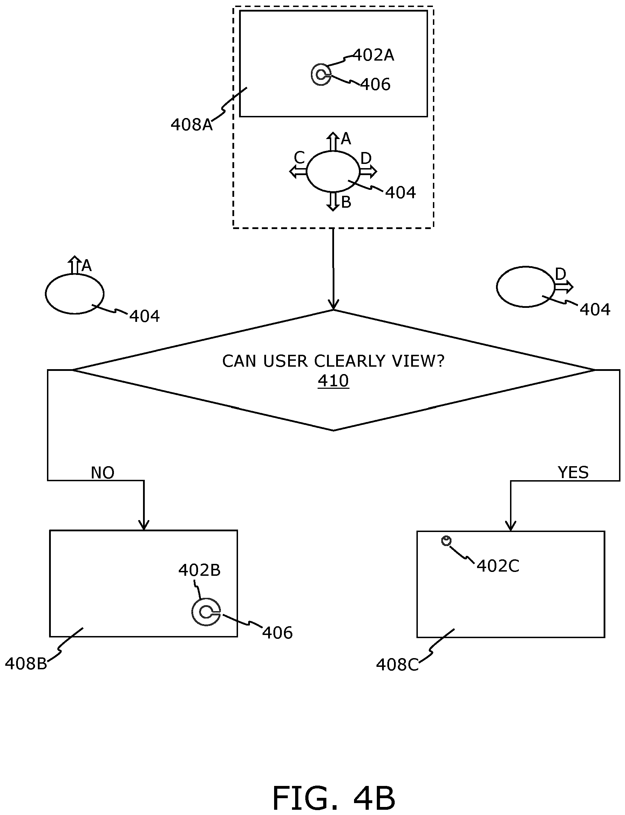

Referring to FIG. 4A, there are shown an exemplary optotype (depicted as a given optotype **402A** in different orientations) and an exemplary input means (depicted as a remote controller **404**), and referring to FIG. 4B, there is shown a flow of an exemplary visual acuity check of a user's eyes is conducted by using the given optotype **402A** and the remote controller **404,** in accordance with an embodiment of the present disclosure.

In both FIGs. 4A and 4B, the given optotype **402A** is depicted as a Landolt C optotype with an optotype feature (depicted as a predefined gap **406**). The input means **404** comprises four buttons, namely, an up-direction button **A** (as shown by an up arrow), a down-direction button **B** (as shown by a down arrow), a left-direction button **C** (as shown by a left arrow), a right-direction button **D** (as shown by a right arrow) which correspond to respective positions of the optotype feature **406** in the given optotype **402A.** Hence, the up-direction button **A** corresponds to a top position of the optotype feature **406,** the down-direction button **B** corresponds to a down position of the optotype feature **406,** the left-direction button **C** corresponds to a left position of the optotype feature **406,** and the right-direction button **D** corresponds to a right position of the optotype feature **406,** in the given optotype **402A.** When the visual acuity check is conducted, the user views the given optotype **402A** while using an optical apparatus.

In FIG. 4B, a given image **408A** is displayed to the user, the given image **408A** representing the given optotype **402A.** The user provides an input using the input means **404,** wherein the input corresponds to a position of the optotype feature **406** as seen by the user. In an instance, the user provides a given user input by pressing the up-direction button **A.** Based on the given user input, it is determined (as shown using a decision box **410**) that the user is not able to clearly view the given optotype **402A** represented in the given image **408A.** When it is determined that the user is not able to clearly view the given optotype **402A,** information is collected indicative of at least one of: a size of the given optotype **402A** that the user is not able to clearly view, a position of the given optotype **402A** and/or the optotype feature **406** in the given image **408A,** an appearance of the features of the user's eyes when the user is not able to clearly view the given optotype **402A** that is displayed at said position. Simultaneously, the visual acuity check is repeated by displaying a next image **408B** on the screen, the next image **408B** representing at least one next optotype (depicted as a next optotype **402B**) whose size is larger than a size of the given optotype **402A** that was represented in the given image **408A.**

In another instance, the user provides a given user input by pressing the right-direction button **D.** Based on the given user input, it is determined (as shown using the decision box **410**) that the user is able to clearly view the given optotype **402A** represented in the given image **408A.** When it is determined that the user is able to clearly view the given optotype **402A,** information is collected indicative of at least one of: a size of the given optotype **402A** that the user is able to clearly view, a position of the given optotype **402A** and/or the optotype feature 406 in the given image **408A,** an appearance of features of the user's eyes when the user is able to clearly view the given optotype **402A** that is displayed at said position. The visual acuity check is repeated by displaying another next image **408C** on the screen, the another next image **408C** representing at least one other next optotype (depicted as other next optotype **402C**) whose size is smaller than a size of the given optotype **402A** that was represented in the given image **408A.**

FIGs. 4A-B are merely examples, which should not unduly limit the scope of the claims herein. A person skilled in the art will recognize many variations, alternatives, and modifications of embodiments of the present disclosure.

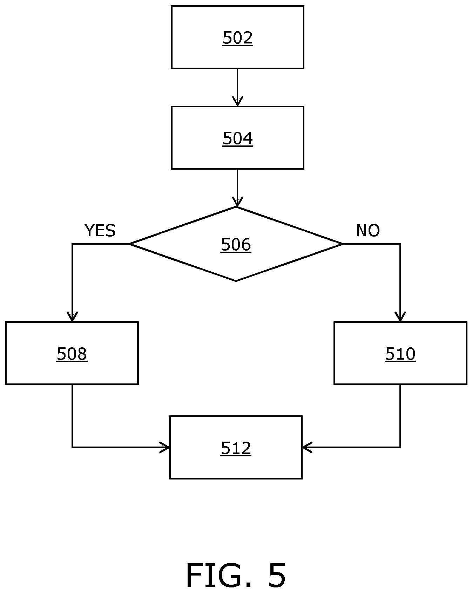

Referring to FIG. 5, illustrated is a flowchart illustrating steps of a method implemented by an optical apparatus comprising an eye-tracking means and at least one processor, wherein the method comprises, in accordance with an embodiment of the present disclosure. At step **502,** a given image is displayed on a screen, the given image representing at least one optotype. At step **504,** a given user input indicative of whether the user is able to clearly view a given optotype represented in the given image is obtained, while using the optical apparatus. At step **506,** based on the given user input, it is determined whether the user is able to clearly view the given optotype represented in the given image. When it is determined that the user is able to clearly view the given optotype, at step **508,** information indicative of at least one of: a size of the given optotype that the user is able to clearly view, a position of the given optotype and/or optotype features in the given image, an appearance of features of the user's eyes when the user is able to clearly view the given optotype that is displayed at said position, is collected. When it is determined that the user is not able to clearly view the given optotype, at step **510,** information indicative of at least one of: a size of the given optotype that the user is not able to clearly view, a position of the given optotype and/or optotype features in the given image, an appearance of the features of the user's eyes when the user is not able to clearly view the given optotype that is displayed at said position, is collected. Additionally, when the user is not able to clearly view the given optotype, the steps **502, 504** and **506** are repeated using a next image, wherein the next image represents at least one next optotype whose size is larger than a size of the given optotype that was represented in the given image. At step **512,** the collected information is processed to determine one or more optical powers specific to the user and to calibrate the eye-tracking means simultaneously.

The aforementioned steps are only illustrative and other alternatives can also be provided where one or more steps are added, one or more steps are removed, or one or more steps are provided in a different sequence without departing from the scope of the claims herein.

## Claims

1. An optical apparatus (102, 200) comprising:
eye-tracking means (108, 204); and
at least one processor (110, 206) configured to:
(a) display a given image (310, 408A) on a screen, the given image representing at least one optotype;
(b) obtain a given user input indicative of whether the user (302) is able to clearly view a given optotype (312, 402A) represented in the given image while using the optical apparatus;
(c) determine, based on the given user input, whether the user is able to clearly view the given optotype represented in the given image;
(d) when it is determined that the user is able to clearly view the given optotype, collect information indicative of at least one of: a size of the given optotype that the user is able to clearly view, a position of the given optotype and/or optotype features (406) in the given image, an appearance of features of the user's eyes (106) when the user is able to clearly view the given optotype that is displayed at said position;
(e) when it is determined that the user is not able to clearly view the given optotype,
collect information indicative of at least one of: a size of the given optotype that the user is not able to clearly view, a position of the given optotype and/or optotype features in the given image, an appearance of the features of the user's eyes when the user is not able to clearly view the given optotype that is displayed at said position; and
repeat steps (a) to (e) using a next image (408B), wherein the next image represents at least one next optotype (402B) whose size is larger than a size of the given optotype that was represented in the given image; and
(f) process the collected information to determine one or more optical powers specific to the user and to calibrate the eye-tracking means simultaneously.

2. The optical apparatus (102, 200) of claim 1, wherein the at least one processor (110, 206) is configured to, when it is determined that the user (302) is able to clearly view the given optotype (312, 402A), repeat steps (a) to (e) using another next image, wherein the another next image (408C) represents at least one other next optotype (402C) whose size is smaller than the size of the given optotype that was represented in the given image (310, 408A).

3. The optical apparatus (102, 200) of any of the preceding claims, further comprises an active optical element (104, 202A-B) per eye (106), wherein the at least one processor (110, 206) is configured to:
control the active optical element to produce at least one of the one or more optical powers;
repeat steps (a) to (e); and
process newly-collected information to further calibrate the eye-tracking means (108, 204).

4. The optical apparatus (102, 200) of claim 3, wherein the at least one processor (110, 206) is configured to adjust the at least one of the one or more optical powers, when it is determined that the user (302) is not able to clearly view a given optotype (312, 402A).

5. The optical apparatus (102, 200) of any of the preceding claims, wherein the at least one optotype comprises at least one of: a Landolt C optotype, a Snellen optotype, a Sloan optotype, an Early Treatment Diabetic Retinopathy Study (ETDRS) optotype, an HOTV letter, an Allen figure, an alphabet character, a number, a symbol, a design, a freeform shape.

6. The optical apparatus (102, 200) of any of the preceding claims, wherein the user (302) views the screen (112, 304) from a predefined distance (D1) lying in a range of 25 centimetres to 750 centimetres.

7. The optical apparatus (102, 200) of any of the preceding claims, wherein a given user input is obtained via at least one input means (114, 210, 306) communicably coupled to the at least one processor (110, 206).

8. The optical apparatus (102, 200) of any of the preceding claims, further comprising:
an active optical element (104, 202A-B) per eye (106);
a frame (208) employed to hold the active optical element per eye; and
at least one input means (114, 210, 306), mounted on a temple of the frame, that is to be used by the user (302) for providing the given user input.

9. The optical apparatus (102, 200) of any of the preceding claims, further comprising an active optical element (104, 202A-B) per eye (106), wherein the at least one processor (110, 206) is further configured to:
process eye-tracking data, collected by the eye-tracking means (108, 204), to determine at least one of: gaze directions of the user's (302) eyes, a gaze point of the user;
determine a given optical depth at which the user is gazing, based on at least one of: an angle of convergence of the gaze directions of the user's eyes, an interpupillary distance of the user's eyes, the gaze point of the user; and
select at least one of the one or more optical powers to be produced by the active optical element, based on the given optical depth at which the user is gazing.

10. A system (100) comprising:
the optical apparatus (102, 200) according to any of the preceding claims;
the screen (112, 304) that is to be employed to display images; and
at least one input means (114, 210, 306) that is to be employed to obtain user input.

11. The system (100) of claim 10, wherein the screen (112, 304) is one of: a display of a device (308) of the user (302) or a medical professional, a projection surface of a projector.

12. A method implemented by an optical apparatus (102, 200) comprising an eye-tracking means (108, 204) and at least one processor (110, 206), wherein the method comprises:
(a) displaying a given image (310, 408A) on a screen (112, 304), the given image representing at least one optotype;
(b) obtaining a given user input indicative of whether the user (302) is able to clearly view a given optotype (312, 402A) represented in the given image while using an optical apparatus according to any of claims 1-9;
(c) determining, based on the given user input, whether the user is able to clearly view the given optotype represented in the given image;
(d) when it is determined that the user is able to clearly view the given optotype, collecting information indicative of at least one of: a size of the given optotype that the user is able to clearly view, a position of the given optotype and/or optotype features (406) in the given image, an appearance of features of the user's eyes (106) when the user is able to clearly view the given optotype that is displayed at said position;
(e) when it is determined that the user is not able to clearly view the given optotype,
collecting information indicative of at least one of: a size of the given optotype that the user is not able to clearly view, a position of the given optotype and/or optotype features in the given image, an appearance of the features of the user's eyes when the user is not able to clearly view the given optotype that is displayed at said position; and
repeating steps (a) to (e) using a next image (408B), wherein the next image represents at least one next optotype (402B) whose size is larger than a size of the given optotype that was represented in the given image; and
(f) processing the collected information to determine one or more optical powers specific to the user and to calibrate the eye-tracking means simultaneously.

13. The method of claim 12, wherein the method comprises when it is determined that the user (302) is able to clearly view the given optotype (312, 402A), repeating steps (a) to (e) using another next image, wherein the another next image (408C) represents at least one other next optotype (402C) whose size is smaller than the size of the given optotype that was represented in the given image (310, 408A).

14. The method of any of claims 12-13, the method comprising:
controlling an active optical element (104, 202A-B) of the optical apparatus (102, 200) to produce at least one of the one or more optical powers;
repeating steps (a) to (e); and
processing newly-collected information to further calibrate the eye-tracking means (108, 204).

15. The method of claim 14, wherein the method comprises adjusting the at least one of the one or more optical powers, when it is determined that the user (302) is not able to clearly view a given optotype (312, 402A).

16. The method of any of claims 12-15, wherein the method further comprises:
processing eye-tracking data, collected by the eye-tracking means (108, 204), to determine at least one of: gaze directions of the user's (302) eyes (106), a gaze point of the user;
determining a given optical depth at which the user is gazing, based on at least one of: an angle of convergence of the gaze directions of the user's eyes, an interpupillary distance of the user's eyes, the gaze point of the user; and
selecting at least one of the one or more optical powers to be produced by an active optical element (104, 202A-B) of the optical apparatus (102, 200), based on the given optical depth at which the user is gazing.
